(19) Europäisches Patentamt
European Patent Office
Office européen des brevets

(11) **EP 3 497 430 B1**

(12) **EUROPÄISCHE PATENTSCHRIFT**

(45) Veröffentlichungstag und Bekanntmachung des Hinweises auf die Patenterteilung:
**06.01.2021 Patentblatt 2021/01**

(21) Anmeldenummer: **17751070.8**

(22) Anmeldetag: **03.08.2017**

(51) Int Cl.:
*G01N 21/3577* *(2014.01)*    *G01N 33/14* *(2006.01)*

(86) Internationale Anmeldenummer:
**PCT/EP2017/069698**

(87) Internationale Veröffentlichungsnummer:
**WO 2018/029088 (15.02.2018 Gazette 2018/07)**

(54) **VERFAHREN ZUM ÜBERPRÜFEN DER ÜBEREINSTIMMUNG EINER BIERPROBE MIT EINEM REFERENZBIER**

METHOD FOR VERIFYING CONFORMITY OF A BEER SAMPLE WITH A REFERENCE BEER

PROCÉDÉ DE VÉRIFICATION DE LA CONFORMITÉ D'UN ÉCHANTILLON DE BIÈRE AVEC UNE BIÈRE DE RÉFÉRENCE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **10.08.2016 DE 102016009636**

(43) Veröffentlichungstag der Anmeldung:
**19.06.2019 Patentblatt 2019/25**

(73) Patentinhaber: **QFood GmbH**
**79194 Gundelfingen (DE)**

(72) Erfinder:
• **KLAPPROTH, Holger**
**79108 Freiburg i. Br. (DE)**
• **SEIDEL, Robert**
**79102 Freiburg i. Br. (DE)**
• **HAAS, Joachim**
**79114 Freiburg (DE)**
• **GREEN, Jonathan E.**
**79194 Gundelfingen (DE)**

(74) Vertreter: **Huwer, Andreas**
**Huwer & Partner**
**Patent- und Rechtsanwälte PartG mbB**
**Schwaighofstrasse 1**
**79100 Freiburg (DE)**

(56) Entgegenhaltungen:
**DE-A1- 10 108 712**

• **FAYOLLE P ET AL: "DETERMINATION OF MAJOR COMPOUNDS OF ALCOHOLIC FERMENTATION BY MIDDEL-INFRARED SPECTROSCOPY: STUDY OF TEMPERATURE EFFECTS AND CALIBRATION METHODS", APPLIED SPECTROSCOPY, THE SOCIETY FOR APPLIED SPECTROSCOPY. BALTIMORE, US, Bd. 50, Nr. 10, 1. Oktober 1996 (1996-10-01), Seiten 1325-1330, XP000642379, ISSN: 0003-7028, DOI: 10.1366/0003702963904872**

**Beschreibung**

Die Erfindung betrifft ein Verfahren zum Überprüfen der Übereinstimmung einer Bierprobe mit einem Referenzbier, welches derselben Biersorte zugeordnet sind wie die Bierprobe, nach dem Oberbegriff von Anspruch 1.

In Brauereibetrieben werden bei der Bierproduktion aufwändige Qualitätskontrollen durchgeführt, die neben einer bakteriellen Untersuchung der hergestellten Biere auch eine sensorische Prüfung der Biere umfassen. Durch die sensorische Prüfung soll sichergestellt werden, dass Biere einer bestimmten, in dem Brauereibetrieb hergestellten Biersorte stets den gleichen, für den Brauereibetrieb typischen Geschmack und Geruch haben. Durch diese Maßnahme sollen die Biere des betreffenden Brauereibetriebs von Wettbewerbsprodukten abgegrenzt und unterschieden werden. Die Bierkonsumenten sollen an den Geschmack gewöhnt und veranlasst werden, ihr Bier bevorzugt bei der betreffenden Brauerei zu beziehen.

In der Praxis erfolgt die sensorische Prüfung der Biere im Rahmen von Bierverkostungen, die in größeren Brauereien von einem geschulten Verkoster-Team durchgeführt werden. Bei der Bierverkostung besteht jedoch das Problem, dass sich das geschmackliche Empfinden der Bierverkoster verändern kann, wenn mehrere Biere hintereinander verkostet werden. Das hängt damit zusammen, dass der Geschmack des Bierverkosters beim Verkosten der ersten Bierprobe noch neutral ist, beim anschließenden Verkosten einer weiteren Bierprobe aber durch die erste Bierprobe verändert sein kann. Dies kann zu Ungenauigkeiten bei der Qualitätsprüfung führen. Ungünstig ist außerdem, dass die sensorische Prüfung der Biere durch Verkosten ziemlich aufwändig ist und dass das Verkoster-Team möglicherweise nicht ständig verfügbar ist.

Aus der DE 101 08 712 A1 ist ein Verfahren zum Überprüfen der Übereinstimmung einer Bierprobe mit einem Referenzbier bekannt, welches derselben Biersorte zugeordnet ist wie die Bierprobe. Von dem Referenzbier werden über den Zeitraum eines Monats ein oder mehrmals täglich Proben gezogen und mittels UV-VIS und NIR Absorptionsspektroskopie vermessen. Es werden Messsignale für das Absorptionsspektrum der einzelnen Referenzbierproben erfasst und für die Messsignale wird eine Hauptkomponentenanalyse durchgeführt wird, bei der für die einzelnen Referenzbierproben jeweils für jede Hauptkomponente eine Faktorladung bestimmt wird. In dem durch die Hauptachsen definierten Koordinatensystem werden die Messwerte der Proben als Punkte dargestellt. Die Messwerte ergeben dabei eine Punktewolke, die mehrere voneinander beabstandete Punkte umfasst. Im Bereich um einen Mittelpunkt herum liegen die Punkte dichter, wohingegen die Punktedichte zum Rand hin abnimmt. Außerdem wird mittels UV-VIS und NIR Absorptionsspektroskopie ein Messsignal für das Absorptionsspektrum der auf Übereinstimmung mit dem Referenzbier zu überprüfenden Bierprobe erfasst. Für die für die Referenzbierproben ermittelten Hauptkomponenten werden die Faktorladungen dieses Messsignals bestimmt und in entsprechenderweise als Punkt in dem durch die Hauptachsen definierten Koordinatensystem dargestellt. Der Punkt der zu überprüfenden Bierprobe wird mit der Punktewolke verglichen und es wird festgestellt, dass der Punkt außerhalb der Punktewolke des Referenzbiers liegt.

Aus Fayoll P. et al: Determination of Major Compounds of Alcoholic Fermentation by Middle-Infrared Spectroscopy: Study of Temperature Effects and Calibration Methods", Applied Spectroscopy, The Society for Applied Spectroscopy, Baltimore, US, Br. 50, Nr. 10, 1. Oktober 1996, Seiten 1325-1330, XP000642379, ISSN: 0003-7028, DOI: 10.1366/0003702963904872 ist ein Verfahren beschrieben, bei dem vier Hauptkomponenten eines durch alkoholische Fermentation hergestellten Getränks mittels Absorptionsspektroskopie im mittleren Infrarotbereich quantitativ ermittelt werden.

Es besteht deshalb die Aufgabe, ein Verfahren der eingangs genannten Art zu schaffen, das es auf einfache und reproduzierbare Weise in einem Brauereibetrieb ermöglicht, die Übereinstimmung einer dort hergestellten Bierprobe mit einem Referenzbier derselben Biersorte zu überprüfen.

Erfindungsgemäß wird diese Aufgabe mit den Merkmalen des Anspruchs 1 gelöst. Diese sehen vor, dass von dem Referenzbier mindestens 15 Referenzbierproben mit den gleichen Zutaten und den gleichen Prozessparametern gebraut wurden, dass mittels Infrarot-Absorptionsspektroskopie Messsignale für das Absorptionsspektrum der einzelnen Referenzbierproben erfasst und für die Messsignale eine Hauptkomponentenanalyse durchgeführt wird, bei der mindestens 15 Hauptkomponenten ermittelt und für die einzelnen Referenzbierproben jeweils für jede Hauptkomponente eine Faktorladung $P_R(i,j)$ bestimmt wird, wobei i die Referenzbierprobe und j die Hauptkomponente bezeichnet, dass aus den Faktorladungen $P_R(i,j)$ für jede Referenzbierprobe und für jede Hauptkomponente jeweils ein Referenzwert

$$R(i,j) = \left| \frac{P_R(i,j) - \mu_P(j)}{\sigma_P(j)} \right|$$

ermittelt wird, wobei $\mu_P(j)$ der Mittelwert aller Faktorladungen der j-ten Hauptkomponente und $\sigma_P(j)$ die Standardabweichung dieser Faktorladungen bedeuten, dass ein Referenzintervall

$$\left[\frac{k}{n}\sum_{i=1}^{n}\sum_{j=1}^{m}R(i,j) - k\sum_{j=1}^{m}\sigma_R(j) \ ... \ \frac{k}{n}\sum_{i=1}^{n}\sum_{j=1}^{m}R(i,j) + k\sum_{j=1}^{m}\sigma_R(j)\right]$$

gebildet wird, wobei n die Anzahl der Referenzbierproben, m die Anzahl der Hauptkomponenten, $\sigma_R(j)$ die Standardabweichung aller Referenzwerte der j-ten Hauptkomponente und k eine Konstante ungleich null ist, dass mittels Infrarot-Absorptionsspektroskopie ein Messsignal für das Absorptionsspektrum der auf Übereinstimmung mit dem Referenzbier zu überprüfenden Bierprobe erfasst wird und für die für die Referenzbierproben ermittelten Hauptkomponenten die Faktorladungen $P_B(i)$ dieses Messsignals bestimmt und aus diesen Faktorladungen $P_B(i)$, den Mittelwerten $\mu_P(j)$ der Faktorladungen der Referenzbierproben für die einzelnen Hauptkomponenten und den Standardabweichungen $\sigma_P(j)$ dieser Faktorladungen ein Kennwert

$$B = k\sum_{j=1}^{m}\left|\frac{P_B(j) - \mu_P(j)}{\sigma_P(j)}\right|$$

gebildet und mit dem Referenzintervall verglichen wird, und dass für den Fall, dass der Kennwert B außerhalb des Referenzintervalls liegt ein Fehler bei der Produktion der Bierprobe angezeigt wird, wobei bei der Infrarot-Absorptionsspektroskopie die Referenzbierproben und die Bierprobe mit Infrarotstrahlung durchleuchtet wird, deren Wellenzahl den Bereich zwischen 950 und 3050 abdeckt.

In vorteilhafter Weise wird das Referenzintervall mit Hilfe von normierten Referenzwerten $R(i,j)$ bestimmt, bei denen der Mittelwert $\mu_P(j)$ aller Faktorladungen der betreffenden Hauptkomponente von der der betreffenden Referenzbierprobe zugeordneten Faktorladung $P_R(i,j)$ subtrahiert und der Betrag dieser Subtraktion durch die Standardabweichung aller Referenzwerte $R(i,j)$ dieser Hauptkomponente dividiert wird. Durch diese Maßnahme gehen Spektralanteile, die durch Bierinhaltsstoffe verursacht sind, die nur in geringer Konzentration in den Referenzbierproben vorliegen, stärker in das Referenzintervall ein als dies der Fall wäre, wenn anstelle der normierten Referenzwerte die Faktorladungen zur Berechnung des Referenzintervalls verwendet würden. Dies ermöglicht einen präzisen Vergleich der Bierprobe mit dem durch die Referenzbierproben repräsentierten Referenzbier.

Die Faktorladungen können mittels einer geeigneten, an sich bekannten Software ermittelt werden. Sie werden auch als "Score" bezeichnet. Die Konstante k kann einen beliebigen Wert ungleich null aufweisen, insbesondere den Wert 1 oder den Kehrwert 1/m der Anzahl m der Hauptkomponenten. Nachdem das Referenzintervall aus den Faktorladungen und der Konstanten ermittelt wurde, kann es beispielsweise zusammen mit den Hauptkomponenten und ggf. der Konstanten k in einem Datenspeicher eines Mikrocomputers abgelegt werden.

Mit den zuvor abgespeicherten Hauptkomponenten, dem Referenzintervall und ggf. dem Faktor k kann das erfindungsgemäße Verfahren auf einfache Weise auf eine Vielzahl von in einer Brauerei hergestellten Bierproben angewendet werden, indem jeweils von der betreffenden Bierprobe mittels eines hochauflösenden Absorptionsspektrometers ein Messsignal für das Absorptionsspektrum aufgenommen wird. Dabei wird dieses Absorptionsspektrum mit den gleichen Parametern erfasst wie die Absorptionsspektren der Referenzbierproben. Vorzugsweise wird für die Messung des Absorptionsspektrums der Bierprobe dasselbe oder zumindest ein baugleiches Absorptionsspektrometer verwendet wie für die Messung der Absorptionsspektren der Referenzbierproben.

Mit Hilfe eines auf dem Mikrocomputer ablaufenden Betriebsprogramms kann aus dem Messsignal und den Hauptkomponenten der Kennwert B ermittelt, mit dem Referenzintervall verglichen und das Ergebnis des Vergleichs zur Anzeige gebracht werden. Falls der Vergleich eine Abweichung ergibt, kann der Braumeister oder ein entsprechender Mitarbeiter der Brauerei bei Bedarf die Qualität der untersuchten Bierprobe durch Verkosten überprüfen und erforderlichenfalls die Produktionsparameter für die Bierherstellung derart verändern, dass der Fehler kompensiert bzw. beseitigt wird.

Der Mikrocomputer kann in das Absorptionsspektrometer oder dessen Steuerung integriert sein. Dies ermöglicht im laufenden Produktionsprozess eine einfache Vorort-Kontrolle der Bierproben. Es ist aber auch möglich, dass der Mikrocomputer von dem Absorptionsspektrometer getrennt und zum Einlesen des Messsignals für das Absorptionsspektrum mit dem Absorptionsspektrometer über eine geeignete Schnittstelle verbindbar ist.

Bei einer bevorzugten Ausführungsform der Erfindung ist die Anzahl n der Referenzbierproben größer oder gleich der Anzahl m der Hauptkomponenten, insbesondere doppelt so groß und bevorzugt mindestens drei Mal so groß wie diese. Durch eine möglichst große Anzahl von Referenzbierproben können durch statistische Schwankungen verursachte Ungenauigkeiten in den Hauptkomponenten weitestgehend vermieden werden.

Bei einer vorteilhaften Ausgestaltung der Erfindung beträgt die Anzahl m der Hauptkomponenten mindestens 20, gegebenenfalls mindestens 30, insbesondere mindestens 40 und bevorzugt mindestens 50. Dies ermöglicht einen sehr präzisen Vergleich der Bierprobe mit dem Referenzbier, wobei auch Abweichungen von Bierinhaltsstoffen berücksichtigt werden können, die nur in sehr geringer Konzentration in der Bierprobe und/oder dem Referenzbier enthalten sind.

Erfindungsgemäß werden bei der Infrarot-Absorptionsspektroskopie die Referenzbierproben und die Bierprobe mit Infrarotstrahlung durchleuchtet, deren Wellenzahl den Bereich zwischen 950 und 3050 abdeckt. In diesem Bereich haben die im Bier enthaltenen Zucker charakteristische Absorptionspeaks, die sich im Spektrum identifizieren lassen. Bei der Messung des Absorptionsspektrums wird eine Flüssigkeitsschicht der Bierprobe bzw. der Referenzbierprobe mit Infrarotstrahlung durchleuchtet. Die Dicke der mit der Infrarotstrahlung durchleuchteten Flüssigkeitsschicht kann maximal 30 μm, gegebenenfalls maximal 20 μm, bevorzugt maximal 15 μm und insbesondere maximal 10 μm betragen.

Nachfolgend wird ein Ausführungsbeispiel der Erfindung näher erläutert.

Bei dem Ausführungsbeispiel werden zum Überprüfen der Übereinstimmung einer zu untersuchenden Bierprobe mit einem Referenzbier, welches derselben Biersorte zugeordnet sind wie die Bierprobe, von dem Referenzbier, 100 Referenzbierproben jeweils mit den gleichen Zutaten und den gleichen Prozessparametern gebraut.

Mittels eines Infrarot-Absorptionsspektrometers vom Typ QFOOD QUANTOS® werden in einem Wellenzahlenbereich, der sich von der Wellenzahl 980 zur Wellenzahl 1200 erstreckt, Messsignale für die Absorptionsspektren der 100 Referenzbierproben erfasst. Jedes Messsignal umfasst jeweils 1000 Wertekombinationen, die jeweils zumindest einen Wert für die Wellenzahl und eine dieser zugeordneten Wert für die optische Infrarot-Absorption der Referenzbierprobe aufweist.

Für die auf diese Weise erfassten 100 Messsignale bzw. Spektren wird eine Hauptkomponentenanalyse durchgeführt, bei der mit Hilfe einer an sich bekannten Software 30 Hauptkomponenten ermittelt werden. Für die 100 Referenzbierproben wird jeweils für jede der 30 Hauptkomponenten eine Faktorladung $P_R(i,j)$ bestimmt. Der Index i bezeichnet die Referenzbierprobe und der Index j die Hauptkomponente. Insgesamt ergeben sich also 3.000 Faktorladungen $P_R(i,j)$, von denen nachstehend aus Gründen der Übersichtlichkeit nur einige dargestellt sind:

|  | j=1 | j=2 | j=3 | ... | j=29 | j=30 |
|---|---|---|---|---|---|---|
| i=1 | -0,1230054 | -0,0026305 | -0,0003498 | ... | -0,00000534 | -0,00000243 |
| i=2 | -0,1242563 | -0,0026599 | -0,0003300 | ... | 0,00000015 | -0,00000127 |
| i=3 | -0,1293215 | -0,0019354 | 0,0000951 | ... | -0,00000968 | 0,00000739 |
| ⋮ | ⋮ | ⋮ | ⋮ | ⋮ | ⋮ | ⋮ |
| i=98 | -0,1294580 | -0,0003328 | 0,00057318 | ... | -0,00000786 | -0,00000556 |
| i=99 | -0,1286656 | -0,0008917 | 0,00045053 | ... | -0,00000487 | -0,00000637 |
| i=100 | -0,1309384 | -0,0004471 | 0,00042969 | ... | -0,00000069 | -0,00000744 |

Für jede der 30 Hauptkomponenten werden jeweils der Mittelwert $\mu_P(j)$ aller Faktorladungen und die Standardabweichung $\sigma_P(j)$ dieser Faktorladungen bestimmt:

| j | $\mu_P(j)$ | $\sigma_P(j)$ |
|---|---|---|
| 1 | -0,12897300 | 0,00196521 |
| 2 | -0,00115020 | 0,00124260 |
| 3 | 0,00052527 | 0,00039921 |
| ⋮ | ⋮ | ⋮ |
| 28 | 0,00000017708 | 0,0000102710 |
| 29 | 0,00000015864 | 0,0000089216 |
| 30 | 0,00000016190 | 0,0000060188 |

Für jede Faktorladung $P_R(i,j)$ wird jeweils entsprechend der Betrags-Formel

$$R(i,j) = \left| \frac{P_R(i,j) - \mu_P(j)}{\sigma_P(j)} \right|$$

ein positiver Referenzwert $R(i,j)$ ermittelt:

| i | R(i, 1) | R(i, 2) | R(i, 3) | ... | R(i, 29) | R(i, 30) |
|---|---|---|---|---|---|---|
| 1 | 3,03662653 | 1,19134758 | 2,19197238 | ... | 0,61632769 | 0,43063337 |
| 2 | 2,40007829 | 1,21500130 | 2,14247494 | ... | 0,00063217 | 0,23790443 |
| 3 | 0,17732606 | 0,63194497 | 1,07755101 | ... | 1,10278655 | 1,20091677 |
| ⋮ | ⋮ | ⋮ | ⋮ | ⋮ | ⋮ | ⋮ |
| 98 | 0,24679333 | 0,65777029 | 0,12001654 | ... | 0,89878767 | 0,95066921 |
| 99 | 0,15641331 | 0,20799952 | 0,18721578 | ... | 0,56364666 | 1,08524718 |
| 100 | 1,00010092 | 0,56582482 | 0,23942857 | ... | 0,09467341 | 1,26302301 |

Außerdem wird für jede Hauptkomponente jeweils die Standardabweichung $\sigma_R(i)$ über alle 100 Referenzwerte der betreffenden Hauptkomponente bestimmt:

| i | $\sigma_R(i)$ |
|---|---|
| 1 | 0,64967043 |
| 2 | 0,54158537 |
| 3 | 0,57910532 |
| ⋮ | ⋮ |
| 28 | 0,60052400 |
| 29 | 0,72734766 |
| 30 | 0,63994051 |

Aus den so erhaltenen Referenzwerten $R(i,j)$ und Standardabweichungen $\sigma_R(i)$ wird entsprechend der Formel

$$\left[\frac{1}{100 \cdot 30}\sum_{i=1}^{100}\sum_{j=1}^{30}R(i,j) - \frac{1}{30}\sum_{j=1}^{30}\sigma_R(j) \quad ... \quad \frac{1}{100 \cdot 30}\sum_{i=1}^{100}\sum_{j=1}^{30}R(i,j) + \frac{1}{30}\sum_{j=1}^{30}\sigma_R(j)\right]$$

ein Referenzintervall gebildet:

$$[21,1333601 - 12,0965087 \; ... \; 21,1333601 + 12,0965087] = [9,0368514 \; ... \; 33,2298688]$$

In einem weiteren Verfahrensschritt wird die auf Übereinstimmung mit dem Referenzbier zu überprüfende Bierprobe bereitgestellt. Mit Hilfe des Infrarot-Absorptionsspektrometers vom Typ QFOOD QUANTOS® wird in einem Wellenzahlenbereich, der mit dem Wellenzahlenbereich, in dem auch die Absorptionsspektren der Referenzbierproben vermessen wurden, ein Messsignal für das Absorptionsspektrum der Bierprobe erfasst.

Für die für die Referenzbierproben ermittelten 30 Hauptkomponenten werden die Faktorladungen $P_B(i)$ dieses Messsignals bestimmt:

| i | $\sigma_R(i)$ |
|---|---|
| 1 | 0,64967043 |
| 2 | 0,54158537 |
| 3 | 0,57910532 |
| ⋮ | ⋮ |
| 28 | 0,60052400 |

(fortgesetzt)

| i | $\sigma_R(i)$ |
|---|---|
| **29** | 0,72734766 |
| **30** | 0,63994051 |

Die so erhaltenen Faktorladungen werden normalisiert, indem jeweils von der betreffenden Faktorladung $P_B(j)$ der Mittelwert $\mu_P(j)$ aller Faktorladungen der Referenzbierproben für die betreffende Hauptkomponente subtrahiert und das Ergebnis dieser Subtraktion durch die Standardabweichung $\sigma_P(j)$ dieser Faktorladungen betragsmäßig dividiert wird:

$$\left| \frac{P_B(j) - \mu_P(j)}{\sigma_P(j)} \right|$$

Aus den so erhaltenen, normalisierten Faktorladungen wird der arithmetische Mittelwert ermittelt, um einen Kennwert B für die Bierprobe zu bilden:

$$B = \frac{1}{30} \sum_{j=1}^{m} \left| \frac{P_B(j) - \mu_P(j)}{\sigma_P(j)} \right| = 52,28199576$$

Dieser Kennwert B wird mit dem Referenzintervall [9,0368514 ... 33,2298688] verglichen. Da der Kennwert B außerhalb des Referenzintervalls liegt, wird ein Fehler bei der Produktion der Bierprobe angezeigt.

**Patentansprüche**

1. Verfahren zum Überprüfen der Übereinstimmung einer Bierprobe mit einem Referenzbier, welches derselben Biersorte zugeordnet ist wie die Bierprobe, wobei von dem Referenzbier mindestens 15 Referenzbierproben mit den gleichen Zutaten und den gleichen Prozessparametern gebraut wurden, wobei mittels Infrarot-Absorptionsspektroskopie Messsignale für das Absorptionsspektrum der einzelnen Referenzbierproben erfasst und für die Messsignale eine Hauptkomponentenanalyse durchgeführt wird, bei der für die einzelnen Referenzbierproben jeweils für jede Hauptkomponente eine Faktorladung $P_B(i,j)$ bestimmt wird, wobei i die Referenzbierprobe und j die Hauptkomponente bezeichnet, und wobei mittels Infrarot-Absorptionsspektroskopie ein Messsignal für das Absorptionsspektrum der auf Übereinstimmung mit dem Referenzbier zu überprüfenden Bierprobe erfasst wird und für die für die Referenzbierproben ermittelten Hauptkomponenten die Faktorladungen $P_S(i)$ dieses Messsignals bestimmt werden, **dadurch gekennzeichnet, dass** bei der Infrarot-Absorptionsspektroskopie die Referenzbierproben und die Bierprobe mit Infrarotstrahlung durchleuchtet werden, deren Wellenzahl den Bereich zwischen 950 und 3050 abdeckt, dass bei der Hauptkomponentenanalyse der Referenzbierproben mindestens 15 Hauptkomponenten ermittelt werden, dass aus den Faktorladungen $P_g(i,j)$ für jede Referenzbierprobe und für jede Hauptkomponente jeweils ein Referenzwert

$$R(i,j) = \left| \frac{P_B(i,j) - \mu_B(j)}{\sigma_B(j)} \right|$$

ermittelt wird, wobei $\mu_P(j)$ der Mittelwert aller Faktorladungen der j-ten Hauptkomponente und $\sigma_P(j)$ die Standardabweichung dieser Faktorladungen bedeuten, dass ein Referenzintervall

$$\left[ \frac{k}{n} \sum_{i=1}^{n} \sum_{j=1}^{m} R(i,j) - k \sum_{j=1}^{m} \sigma_R(j) \quad \dots \quad \frac{k}{n} \sum_{i=1}^{n} \sum_{j=1}^{m} R(i,j) + k \sum_{j=1}^{m} \sigma_R(j) \right]$$

gebildet wird, wobei n die Anzahl der Referenzbierproben, m die Anzahl der Hauptkomponenten, $\sigma_P(j)$ die Standardabweichung aller Referenzwerte der j-ten Hauptkomponente und k eine Konstante ungleich null ist, und aus den Faktorladungen $P_S(i)$ des Messsignals für das Absorptionsspektrum der auf Übereinstimmung mit dem Referenzbier zu überprüfenden Bierprobe, den Mittelwerten $\mu_P(j)$ der Faktorladungen der Referenzbierproben für die einzelnen Hauptkomponenten und den Standardabweichungen $\sigma_P(j)$ dieser Faktorladungen ein Kennwert

$$B = k \sum_{i=1}^{m} \left| \frac{P_E(j) - \mu_E(j)}{\sigma_E(j)} \right|$$

gebildet und mit dem Referenzintervall verglichen wird, und dass für den Fall, dass der Kennwert B außerhalb des Referenzintervalls liegt ein Fehler bei der Produktion der Bierprobe angezeigt wird.

2. Verfahren nach Anspruch 1, **dadurch gekennzeichnet, dass** die Anzahl n der Referenzbierproben größer oder gleich der Anzahl m der Hauptkomponenten ist, insbesondere doppelt so groß und bevorzugt mindestens drei Mal so groß ist wie diese.

3. Verfahren nach Anspruch 1 oder 2, **dadurch gekennzeichnet, dass** die Anzahl m der Hauptkomponenten mindestens 20, gegebenenfalls mindestens 30, insbesondere mindestens 40 und bevorzugt mindestens 50 beträgt.

4. Verfahren nach einem der Ansprüche 1 bis 3, **dadurch gekennzeichnet, dass** die Konstante k dem Kehrwert der Anzahl m der Hauptkomponenten entspricht.

**Claims**

1. Method of verifying the conformance of a beer sample with a reference beer attributed to the same beer type as the beer sample, wherein at least 15 reference beer samples of the reference beer are brewed with the same ingredients and the same process parameters, with detection of measurement signals for the absorption spectrum of the individual reference beer samples by means of infrared absorption spectroscopy and performance of a main component analysis for the measurement signals in which a factor load $P_R(i,j)$ is determined for each main component for the individual reference beer samples, where i denotes the reference beer sample and j the main component, and wherein a measurement signal for the absorption spectrum of the beer sample to be verified for conformance with the reference beer is detected by means of infrared absorption spectroscopy and the factor loads $P_B(i)$ of this measurement signal are determined for the main components ascertained for the reference beer samples, **characterized in that** the infrared absorption spectroscopy involves passing infrared radiation having a wavenumber covering the range between 950 and 3050 through the reference beer samples and the beer sample, **in that** at least 15 main components are ascertained in the main component analysis of the reference beer samples, **in that** the factor loads $P_R(i,j)$ for each reference beer sample and for each main component are used to ascertain a reference value

$$R(i,j) = \left| \frac{P_R(i,j) - \mu_P(j)}{\sigma_P(j)} \right|$$

where $\mu_P(j)$ denotes the average of all factor loads of the jth component and $\sigma_P(j)$ the standard deviation of these factor loads, **in that** a reference interval

$$\left[ \frac{k}{n} \sum_{i=1}^{n} \sum_{j=1}^{m} R(i,j) - k \sum_{j=1}^{m} \sigma_R(j) \ ... \ \frac{k}{n} \sum_{i=1}^{n} \sum_{j=1}^{m} R(i,j) + k \sum_{i=1}^{m} \sigma_R(j) \right]$$

is formed, where n is the number of reference beer samples, m is the number of main components, $\sigma_R(j)$ is the standard deviation of all reference values of the jth main component and k is a non-zero constant, and the factor loads $P_B(i)$ of the measurement signal for the absorption spectrum of the beer sample to be verified for conformance with the reference beer, the averages $\mu_P(j)$ of the factor loads of the reference beer samples for the individual main components and the standard deviations $\sigma_P(j)$ of these factor loads are used to form a characteristic value

$$B = k \sum_{j=1}^{m} \left| \frac{P_B(j) - \mu_P(j)}{\sigma_P(j)} \right|$$

which is compared with the reference interval and, if the characteristic value B is outside the reference interval, a fault in the production of the beer sample is indicated.

2. Method according to Claim 1, **characterized in that** the number n of reference beer samples is not less than the number m of main components, and especially twice as high and preferably three times as high as the latter.

3. Method according to Claim 1 or 2, **characterized in that** the number m of main components is at least 20, optionally at least 30, especially at least 40 and preferably at least 50.

4. Method according to any of Claims 1 to 3, **characterized in that** the constant k corresponds to the reciprocal of the number m of main components.

**Revendications**

1. Procédé de vérification de la concordance d'un échantillon de bière avec une bière de référence qui est associée au même type de bière que l'échantillon de bière, au moins 15 échantillons de la bière de référence ayant été brassés avec les mêmes ingrédients et les mêmes paramètres de processus, des signaux de mesure du spectre d'absorption des échantillons de bière de référence individuels étant détectés au moyen d'une spectroscopie d'absorption infrarouge et une analyse des composants principaux étant effectuée pour les signaux de mesure, analyse lors de laquelle une charge factorielle $P_a(i,j)$ est déterminée pour chaque composant principal des échantillons de bière de référence individuels, i désignant l'échantillon de bière de référence et j désignant le composant principal, et un signal de mesure du spectre d'absorption de l'échantillon de bière dont la concordance avec la bière de référence doit être vérifiée étant détecté au moyen d'une spectroscopie d'absorption infrarouge et les charges factorielles $P_a(i,j)$ de ce signal de mesure étant déterminées pour les composants principaux déterminés pour les échantillons de bière de référence, **caractérisé en ce que**, lors de la spectroscopie d'absorption infrarouge, les échantillons de bière de référence et l'échantillon de bière sont illuminés avec un rayonnement infrarouge dont le nombre d'onde couvre la gamme comprise entre 950 et 3050, **en ce qu'**au moins 15 composants principaux sont déterminés lors de l'analyse des composants principaux des échantillons de bière de référence, **en ce qu'**une valeur de référence

$$R(i,j) = \left| \frac{P_R(i,j) - \mu_P(j)}{\sigma_P(j)} \right|$$

est déterminée à partir des charges factorielles $P_a(i,j)$ pour chaque échantillon de bière de référence et pour chaque composant principal, où $\mu_P(i)$ désigne la valeur moyenne de toutes les charges factorielles du j-ième composant principal et $\sigma_P(j)$ désigne l'écart-type de ces charges factorielles, **en ce qu'**un intervalle de référence est formé

$$\left[ \frac{k}{n} \sum_{i=1}^{n} \sum_{j=1}^{m} R(i,j) - k \sum_{j=1}^{m} \sigma_R(j) \dots \frac{k}{n} \sum_{i=1}^{n} \sum_{j=1}^{m} R(i,j) + k \sum_{j=1}^{m} \sigma_R(j) \right]$$

où n est le nombre d'échantillons de bière de référence, m est le nombre de composants principaux, $\sigma_R(j)$ est l'écart-type de toutes les valeurs de référence du j-ième composant principal et k est une constante non nulle, et une valeur caractéristique

$$B = k \sum_{j=1}^{m} \left| \frac{P_B(j) - \mu_P(j)}{\sigma_P(j)} \right|$$

est formée à partir des charges factorielles $P_B(i)$ du signal de mesure du spectre d'absorption de l'échantillon de bière dont la concordance avec la bière de référence doit être vérifiée, des valeurs moyennes $\mu_P(j)$ des charges factorielles des échantillons de bière de référence pour les composants principaux individuels et des écarts-types $\sigma_P(j)$ de ces charges factorielles, et est comparée à l'intervalle de référence, et **en ce que**, dans le cas où la valeur caractéristique B est située en dehors de l'intervalle de référence, une erreur est indiquée lors de la production de l'échantillon de bière.

2. Procédé selon la revendication 1, **caractérisé en ce que** le nombre n d'échantillons de bière de référence est supérieur ou égal au nombre m de composants principaux, notamment deux fois supérieur et de préférence au moins trois fois supérieur à celui-ci.

3. Procédé selon la revendication 1 ou 2, **caractérisé en ce que** le nombre m de composants principaux est d'au moins 20, éventuellement d'au moins 30, en particulier d'au moins 40 et de préférence d'au moins 50.

4. Procédé selon l'une des revendications 1 à 3, **caractérisé en ce que** la constante k correspond à l'inverse du nombre m de composants principaux.

**IN DER BESCHREIBUNG AUFGEFÜHRTE DOKUMENTE**

**In der Beschreibung aufgeführte Patentdokumente**

- DE 10108712 A1

**In der Beschreibung aufgeführte Nicht-Patentliteratur**

- Determination of Major Compounds of Alcoholic Fermentation by Middle-Infrared Spectroscopy: Study of Temperature Effects and Calibration Methods. **FAYOLL P. et al.** Applied Spectroscopy. The Society for Applied Spectroscopy, 01. Oktober 1996, 1325-1330